# FASCICULE DE BREVET EUROPEEN

(11) **EP 4 010 110 B1**
(45) Date de publication et mention de la délivrance du brevet: **27.11.2024**
(21) Numéro de dépôt: 20753997.4
(22) Date de dépôt: 20.07.2020
(51) Int. Cl.: B01J 13/10, B01J 13/14, A23L 27/00, A23P 10/30, A61K 8/11, A61K 9/50, C09B 67/02, C11D 3/50, A01N 25/28

(54) **PROCÉDÉ DE FABRICATION DE MICROCAPSULES RENFERMANT UN ACTIF LIPOPHILE, MICROCAPSULES PRÉPARÉES PAR CE PROCÉDÉ ET LEUR UTILISATION**
VERFAHREN ZUR HERSTELLUNG VON MIKROKAPSELN, DIE EINEN LIPOPHILEN WIRKSTOFF ENTHALTEN, DURCH DAS VERFAHREN HERGESTELLTE MIKROKAPSELN UND IHRE VERWENDUNG
METHOD FOR MANUFACTURING MICROCAPSULES CONTAINING A LIPOPHILIC ACTIVE INGREDIENT, MICROCAPSULES PREPARED BY SAID METHOD AND THE USE THEREOF

(30) Priorité: 06.08.2019 FR 1908996
(43) Date de publication de la demande: 15.06.2022
(73) Titulaire: Microcapsules Technologies, 45390 Puiseaux (FR)
(72) Inventeur: HABAR, Gérard, 77780 BOURRON-MARLOTTE (FR)
(74) Mandataire: Ipsilon
(86) Numéro de dépôt international: PCT/FR2020/051302
(87) Numéro de publication internationale: WO 2021/023922

(56) Documents cités:
- EP-B1- 3 092 069
- WO-A1-2008/009216
- WO-A1-2018/172514

## Description

### DOMAINE DE L'INVENTION

La présente invention concerne le domaine des procédés de fabrication des microcapsules de type microcapsules à réservoir, les microcapsules ainsi réalisées et leur utilisation dans des formulations telles que des lessives ou des produits cosmétiques.

Les microcapsules appelées microcapsules à réservoir (ou encore dénommées microcapsules core/shell) sont des microcapsules du type renfermant un principe actif dans une enveloppe à base de polymère.

Les procédés de fabrication de ces microcapsules, et donc d'incorporation d'un principe actif lipophile dans un polymère, comprennent les étapes consistant à :
- disperser au moins un principe actif lipophile dans une phase continue aqueuse, de manière à former une émulsion ou une dispersion de gouttelettes de type huile dans l'eau,
- polymériser in situ un précurseur du polymère à la périphérie desdites gouttelettes pour former la paroi de l'enveloppe des microcapsules, en enfermant le principe actif.

### ETAT DE LA TECHNIQUE

Cette micro-encapsulation peut être réalisée suivant deux grandes voies principales :
- Une des premières voies met en oeuvre un procédé de coacervation complexe à partir de polymères d'origine naturelle tels que la gélatine, comme décrit dans le brevet EP 0 674 942 B1. Ce brevet porte plus spécifiquement sur la micro-encapsulation de substances chromogènes hydrophobes, les microcapsules étant destinées à être introduites dans une composition de revêtement pour papier sensible à la pression. De telles microcapsules ne sont pas appropriées pour résister en présence de tensio-actifs, tels qu'en milieu détergent.
- Une seconde voie concerne des procédés impliquant la formation de copolymères synthétiques, parmi lesquels on peut citer la polymérisation de monomères ou de oligomères organiques aminés en présence d'aldéhyde(s), utilisant notamment des résines mélamine/formol ou mettant en oeuvre des monomères de type silicates ou silicone pour réaliser l'enveloppe des microcapsules, qui conduisent à des microcapsules étanches mais ne résistant pas en présence de détergents. Pour pallier ces inconvénients a été proposé plus récemment dans le brevet EP 3 092 069 B1 un procédé de fabrication de microcapsules contenant un actif lipophile dont l'enveloppe à double paroi est formée de deux polymères l'un étant un copolymère de silicone, l'autre un polymère organique aminé, résistante aux tensio-actifs.

### BUTS DE L'INVENTION

Le propriétaire a souhaité s'affranchir, au moins partiellement, de l'utilisation de polymères synthétiques, pour améliorer la biodégradabilité des microcapsules, tout en conservant leur étanchéité et leur aptitude de résistance en présence de tensio-actifs.

Un premier but de l'invention est donc de proposer un procédé de fabrication de microcapsules à réservoir incorporant un actif lipophile mettant en oeuvre des matières premières d'origine naturelle, telles que des substances d'origine animale ou végétale.

Un autre but de l'invention est de proposer un procédé de fabrication de microcapsules à réservoir étanches, et résistantes aux agents tensio-actifs, en vue d'une utilisation dans des compositions détergentes ou cosmétiques.

Un autre but de l'invention est de proposer un procédé de fabrication de microcapsules à réservoir présentant un taux de formaldéhyde nul.

### RESUME DE L'INVENTION

A cet effet la présente invention concerne un procédé de fabrication de microcapsules à réservoir, renfermant un principe actif lipophile dans une enveloppe formant la paroi desdites microcapsules.

Selon l'invention le procédé comprend les étapes suivantes :
(i) ajout à une phase lipophile, renfermant au moins un principe actif, d'au moins un monomère ou oligomère silane et/ou de silicates, d'au moins une résine mélamine et d'au moins un isocyanate, pour former un mélange A,
(ii) dispersion sous agitation du mélange A obtenu à l'étape (i) dans une phase continue aqueuse B à pH acide renfermant au moins une gélatine et/ou au moins une protéine végétale hydrosoluble, et au moins un polyacide, de manière à former par coacervation complexe une émulsion ou une dispersion de gouttelettes de type huile dans l'eau, enveloppées d'un coacervat, et à initier la formation d'un premier copolymère silicone/mélamine/polyuréthane enfermant ledit principe actif,
(iii) introduction éventuelle d'un colloïde protecteur de type dérivé de cellulose dans la phase continue aqueuse contenant la dispersion de gouttelettes,
(iv) ajout, à la dispersion de gouttelettes, à une température inférieure ou égale à 10°C, d'au moins un agent de réticulation du coacervat, permettant la co-réticulation, à l'interface des dites gouttelettes, du coacervat à base de gélatine et/ou de protéine végétale avec le premier copolymère silicone/mélamine/polyuréthane en formation, conduisant à des polymères co-réticulés, constituant la paroi desdites microcapsules en enfermant ledit principe actif,
(v) obtention d'une suspension aqueuse de microcapsules enfermant le principe actif lipophile.

Le polyacide de la phase aqueuse B comprend avantageusement un polyacide de type poly(méth)acrylique ou polyaspartique et une carboxyalkylcellulose, de préférence la carboxyméthylcellulose.

Le coacervat est le complexe formé alors par l'association de la gélatine ou protéine chargée positivement en milieu acide avec l'acide poly(méth)acrylique ou polyaspartique et la carboxyméthylcellulose chargés négativement.

Les principaux avantages du procédé selon la présente invention est de permettre la fabrication de microcapsules « à réservoir » à partir de monomères bon marché disponibles facilement en ce qui concerne le copolymère silicone/mélamine/polyuréthane et d'utiliser un polymère organique biodégradable (à base de gélatine et/ou protéine végétale).

L'intérêt de ce nouveau procédé d'encapsulation est aussi de combiner les propriétés des membranes de silicone très réticulées à celle des membranes organiques de façon à obtenir un effet barrière sur mesure, et également d'assurer à l'ensemble de la structure de la microcapsule de bonnes performances mécaniques grâce aux réactions chimiques covalentes liant les deux types de polymères.

Ainsi l'enveloppe formant la paroi desdites microcapsules est à la fois biodégradable et résistante aux tensio-actifs.

### DESCRIPTION DETAILLEE DE L'INVENTION

De manière avantageuse les proportions pondérales des constituants destinés à former le copolymère silicone/mélamine/polyuréthane, introduits dans le mélange A sont respectivement de 50 à 80% du monomère ou oligomère silane et/ou silicate, de 25 à 10% de la résine mélamine et de 25 à 10% de l'isocyanate, exprimées en poids sec du poids total desdits constituants.

La phase aqueuse B renferme de préférence les proportions pondérales suivantes : 50 à 80 % de gélatine et/ou de protéine végétale, 10 à 30 % de carboxyalkylcellulose, et 5 à 20 % de polyacide de type poly(méth)acrylique ou polyaspartique, exprimées en % du poids sec de coacervat.

Le ou les composé(s) silanes et/ou silicates est/sont de préférence choisis parmi les composés de formule (I) ou (II) ci-après : 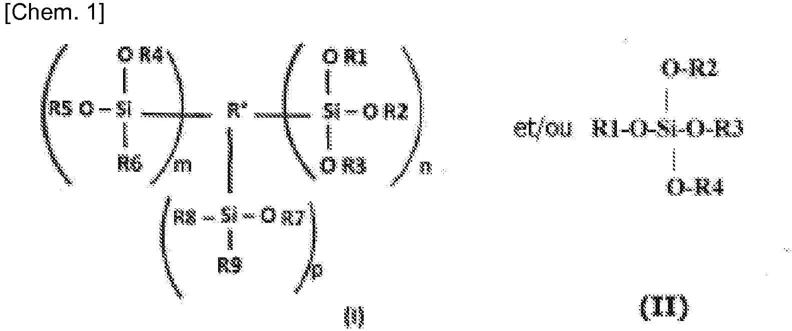
dans lesquels R1, R2, R3, R4, R5, R6, R7, R8, R9 sont des radicaux alkyle,
linéaires ou cycliques, substitués ou non,
R° est une molécule organique et/ou silicone,
les groupes entre ( ) étant reliés à R° par un atome de silicium et sont présents m, n, ou p fois, et m, n, p pouvant être nuis individuellement, mais la somme m+n+p étant au moins égale à 1.

La valeur m+n+p qui est au minimum de 1 n'est pas limitée théoriquement, mais pratiquement par la viscosité du produit. Le polymère de silicone très réticulé utilisé ici apporte son hydrophobie et renforce la structure de la paroi des microcapsules.

Le monomère ou oligomère silane peut, par exemple, être choisi parmi les composés ci-après :
les trialkoxysilanes de formule (I) dans laquelle m et p sont nuls : R-Si (OR1)(OR2)(OR3) dans lequel R représente un radical alkyl à 1 à 20 atomes de carbone, substitué ou non choisi parmi les groupes méthyl, éthyl, n-propyl, isopropyl, 1-n-butyl, 2-n-butyl, isobutyl, tert-butyl, n-pentyl, isopentyl, neopentyl, tert-pentyl, hexyl, heptyl, octyl tel que le n-octyl ou l'isooctyl, 2,2,4-triméthylpentyl, nonyl, decyl, dodecyl, octadecyl , cycloalkyl tel que cyclopentyl, cyclohexyl et cycloheptyl et methylcyclohexyl, aryl tel que les phényl, naphthyl, anthryl et phénanthryl, alkaryl tel que les o-, m- and p-tolyl, xylyl et éthylphényl, et aralkyl tel que les benzyl, alpha.-et beta.-phényléthyl,
R pouvant également être halogéné, tel que les 3,3,3-trifluoro-n-propyle, 2,2,2,2',2',2'-hexafluoroisopropyle, heptafluoroisopropyl, o-, m- and p-chlorophényles..

R pouvant être insaturé tel que les vinyl, 5-hexenyl, 2,4-divinylcyclohexyléthyl, 2-propenyl, allyl, 3-butenyl and 4-pentenyl, éthynyl, propargyl and 2-propynyl.

R1, R2 et R3 pouvant être identiques ou différents, sont par exemple choisis parmi les radicaux méthyl ou éthyl, ou peuvent être oxygénés tels les méthoxyéthyle, éthoxyéthyle, acétoxy, ou oxymino.

A titre d'exemples non limitatifs sont cités quelques monomères intéressants parmi lesquels :
- Le chloropropylméthyldiméthoxysilane, le gamma.-mercaptopropyltriméthoxy ou éthoxy silane, le gamma.-isocyanate propyltriéthoxysilane,
- Les époxydes : le glycidoxypropyl triméthoxy ou triéthoxy silane, le glycidoxy propylméthyldiéthoxysilane, le (3,4-époxycyclohexyl)éthyltriméthoxy ou triéthoxy silane,
- Les silanes acryliques : l'acryloxypropyltriméthoxysilane, le méthacryloxypropyl)triméthoxysilane, le gamma.-méthacryloxypropylméthyldiméthoxy ou diéthoxy silane,
- Les silanes porteurs d'atomes de soufre : le gamma.-mercaptopropyltriméthoxysilane, le mercaptopropylmethyldimethoxysilane, le Bis-{3-(triéthoxysilyl)propyl}polysulfure, le Bis-{3-(triéthoxysilyl)propyl}disulfure, le 3-octanoylthio-1-propyltriéthoxysilane. En effet les silanes portant des groupes thiols réagissent facilement avec les groupes isocyanates, conduisant à des polythiouréthanes qui ont un intérêt évident ici.
- Les silanes aminés : le 3-aminopropyltriéthoxy ou méthoxysilane, le N-(nbutyl)-3-aminopropyltriméthoxy ou éthoxy silane, le N-aminoéthyl-3-aminopropylméthyldiméthoxysilane, le N-aminoéthyl-3-aminopropyltriméthoxy ou triéthoxysilane, le 3-aminopropylméthyldiéthoxysilane, le N-phénylaminopropyl triméthoxysilane, le 2-aminoéthylaminopropyltriméthoxysilane, le 2-aminoéthylaminopropylméthyldimethoxysilane, l'anilinopropyltriméthoxysilane, le gamma.-[N-(.beta.-aminoéthyl)amino]propylméthyldiméthoxysilane, le 4-amino-3,3-diméthylbutyltriméthoxysilane, le 4-amino-3,3-diméthylbutylméthyldiméthoxysilane, le Bis-{gamma-(triméthoxysilyl)propyl}amine, le N-éthyl-gamma-aminoisobutyl triméthoxysilane, le 3-uréidopropyltriéthoxysilane, l'hexaméthyldisilazane, l'alkylene oxydetriméthoxysilane, le Tris-{3-(triméthoxysilyl)propyl}isocyanurate, le Bis(triéthoxysilyl)éthane,
- Des monomères monoalkoxysilane ou dialkoxysilane destinés à diminuer le degré de réticulation et ainsi assouplir le polymère de silicone.
- Des monomères et pré-polymères de type ester siliciques Si (OR')4, dans lesquels R' est identique aux R' décrits précédemment.

Il est bien entendu possible d'utiliser des monomères plus complexes tels que les tris alkoxy isocyanurates ou les bis alkoxy isocyanurates par exemple, ainsi que les oligomères des produits présentés ci-dessus.

Sont également utilisables des silanes chlorés du type R-Si Cl₃, R-Si-Cl₂R' ou encore R-Si-CI R' R".

De manière avantageuse, le composé de formule (II) est choisi parmi le polysilicate de méthyle, le polysilicate d'éthyle ou un mélange de ceux-ci.

La mélamine est de préférence choisie parmi une mélamine-aldéhyde ou une mélamine-carbamate liposoluble, et est de préférence une mélamine-carbamate. La résine mélamine-carbamate est une résine qui agit comme les mélamines-formol tout en offrant notamment un taux de formaldéhyde nul et une bonne solubilité dans les parfums. Il est à noter que cette résine réagit sur les groupes OH et autres présents dans le coacervat et ainsi renforce la structure. Ceci est avantageux dans le procédé de la présente invention, car la présence d'une résine mélamine dans l'eau perturberait tout le système. Etonnamment le butanol ou un alcool différent utilisé pour former et dissoudre la résine mélamine, libéré lors de la réaction de ce polymère avec les autres, ne perturbe pas le bon déroulement de la coacervation complexe.

L'isocyanate utilisé dans le mélange A de l'étape (i) du procédé selon l'invention est de préférence choisi parmi : les diisocyanates de toluène TDI, d'hexaméthylène HDI, de diphénylméthylène MDI, ou d'isophorone IPDI, les dimères ou trimères du diisocyanate d'hexaméthylène tel que l'isocyanurate d'hexaméthylène, les uretdione, ou uretonimine, ou plusieurs de ceux-ci. Les dérivés de HDI sont cependant préférés pour leur tenue aux UV et leur meilleure hydrophilie. Parmi les dérivés de HDI l'isocyanurate est intéressant en raison de sa faible viscosité, son absence de volatilité et également sa solubilité. Sa réactivité est également adaptée. Il est à noter que les isocyanates réagissent également sur les groupes OH, NH et NH₂ ainsi que les SH présents dans le coacervat et ainsi renforcent la structure de la paroi.

Le propriétaire a aussi constaté que les groupes isocyanates ainsi que la résine mélamine permettent de réagir sur tous les groupes réactifs présents et ainsi de réticuler tout l'édifice. Ceci présente l'intérêt d'améliorer considérablement la tenue des microcapsules par rapport à celles obtenues par coacervation complexe uniquement dans les produits lessiviels à des températures voisines de 40°C.

Il est remarquable, et tout à fait surprenant pour l'homme de l'art, qu'aucun des produits utilisés ici ne perturbe la coacervation complexe qui est une opération plutôt délicate.

L'étape (ii) du procédé est avantageusement effectuée à un pH compris entre 3,0 et 5,5, de préférence compris entre 3,5 et 4,5, par ajout dans la phase aqueuse d'au moins un acide comprenant l'acide nitrique. De manière surprenante l'acide nitrique qui n'est que très rarement utilisé en coacervation complexe traditionnelle s'est révélé être un bon catalyseur, en respectant les intervalles de pH ci-dessus, de polymérisation pour le copolymère silicone/mélamine/polyuréthane et un bon agent de formation du coacervat. En variante les acides chlorhydrique, acétique, sulfurique, oxalique, formique sont également utilisables.

Selon une variante du procédé de la présente invention l'étape (ii) peut être réalisée en deux temps :
- dans un premier temps dispersion du mélange A obtenu à l'étape (i) dans une phase continue aqueuse B à pH acide renfermant au moins une gélatine et/ou au moins une protéine végétale hydrosoluble, au moins un polyacide de type poly(méth)acrylique,
- puis dans un second temps ajout d'une solution aqueuse de carboxyalkylcellulose à la dispersion obtenue dans le premier temps.

L'origine de la gélatine n'est pas fondamentale. Celle-ci peut être une gélatine de peau de porc, de poisson ou autre. Il est même possible de remplacer tout ou partie de cette gélatine par une protéine végétale sélectionnée pour sa solubilité dans l'eau. Ce dernier point est important pour les marchés de l'industrie cosmétique et pharmaceutique pour lesquels les produits d'origine animale sont très peu tolérés. Des protéines de blé, de soja ou d'autres céréales ou des hydrolysats de ces végétaux peuvent être utilisés par exemple ici.

L'agent de fixation et de réticulation introduit à l'étape (iv) comprend le glutaraldéhyde.

De manière avantageuse, les gammes de températures des différentes étapes du procédé de fabrication des microcapsules sont les suivantes : l'étape (i) est réalisée à température ambiante (15-25 °C), l'étape (ii) à une température comprise entre 40° et 50 °C, l'émulsion formée étant ensuite refroidie à une température comprise entre 7°C et 10 °C, puis est ajouté le glutaraldéhyde et cette température est maintenue pendant au moins 4 heures, avant que ne soit terminée la polymérisation à chaud entre 40°C et 80°C pendant 1 à 6h.

En résumé sur les étapes du procédé selon l'invention, on prépare tout d'abord le copolymère dit intérieur : les ingrédients devant former le polymère silicone/mélamine/polyuréthane, soit le polymère intérieur, sont dissous à froid (température ambiante) dans le parfum ou autre phase interne lipophile par simple agitation pour former le mélange A. Sont ainsi introduits successivement le ou les précurseur(s) de silicone, la ou les résine(s) mélamine et l'isocyanate ou les isocyanates. De préférence, les molécules les moins solubles et/ou les moins réactives sont dissoutes en premier, en terminant par les plus réactives.

Une fois la phase interne prête, on procède alors rapidement à l'encapsulation proprement dite pour éviter les réactions de polymérisation prématurées du polymère provenant de la phase interne.

Le mélange A est alors dispersé dans de l'eau contenant les polymères destinés à la coacervation complexe.

Selon une première variante de l'invention, la gélatine ou la protéine végétale est dissoute dans de l'eau, à laquelle est ajouté le polyacide de type poly(méth)acrylique ou polyaspartique ainsi que l'acide nitrique abaissant le pH. La solution organique formant le mélange A préalablement préparée est alors introduite dans le mélange aqueux et émulsifiée, en présence ou non de colloïde protecteur (ce colloïde protecteur étant de préférence non ionisé), pour former par coacervation complexe un polymère se déposant autour des gouttelettes produisant ainsi une émulsion ou une dispersion de type huile dans l'eau. L'ensemble est ensuite versé dans une solution de carboxyalkylcellulose préparée préalablement.

Selon une seconde variante de l'invention, la gélatine ou la protéine végétale est dissoute dans de l'eau, à laquelle sont ajoutés le polyacide de type poly(méth)acrylique ou polyaspartique et la carboxyalkylcellulose. La solution organique formant le mélange A préalablement préparée est alors introduite dans le mélange aqueux et émulsifiée, en présence ou non de colloïde protecteur de préférence non ionisé. Pour initier la coacervation complexe on abaisse le pH par ajout d'acide, d'environ 6 jusqu'à environ 4,5, le coacervat se déposant alors sur les gouttelettes et conduisant ainsi à une émulsion ou une dispersion de type huile dans l'eau.

L'émulsion préparée à une température ne dépassant pas 50°C est ensuite refroidie à environ 8°C, le glutaraldéhyde est introduit avec éventuellement d'autres réticulants, On laisse l'ensemble à froid plusieurs heures avant de remonter la température et de finir de polymériser le tout plusieurs heures à plus haute température.

Enfin on termine les opérations en revenant à la température ambiante. Une suspension aqueuse de microcapsules est ainsi obtenue.

La formation de l'émulsion et le maintien de son intégrité au cours de l'encapsulation est favorisé par l'introduction d'un polymère hydrosoluble dans la phase aqueuse continue, dit colloïde protecteur. Ces produits bien connus des praticiens, peuvent être par exemple des dérivés de cellulose tel que hydroxyéthylcellulose, la méthylcellulose, les polyvinylpyrrolidone et les copolymères de polyvinylpyrrolidone, les alcools polyviniliques plus ou moins hydrolysés ainsi que leur copolymères, des polymères d'origine naturelle comme la gomme de xanthane, les alginates, pectines, amidons et dérivés, la caséine, en évitant les polymères trop ionisés susceptibles de perturber la coacervation complexe.

Divers catalyseurs métalliques ou organométalliques peuvent être utilisés pour compléter la réaction de polymérisation. Il peut s'agir par exemple de composés renfermant de l'étain tels le dilaurate ou le diacétate de dibutylétain, l'octoate d'étain, les sels minéraux d'étain, des composés de platine, de zinc, de zirconium, d'aluminium, de titane dont les titanates, ou encore des fluorures, cette liste n'étant pas limitative.

Les actifs lipophiles pouvant être encapsulés selon le procédé de la présente l'invention sont très nombreux, la seule limitation étant qu'ils supportent les conditions de température et de pH des étapes d'encapsulation et qu'ils soient suffisamment solvants pour être capables de dissoudre les produits réactifs introduits dans la phase interne.

Nous citerons parmi les actifs intéressants les acides et alcools gras, les solvants organiques, les hydrocarbures, les esters, les fluides et gommes silicone, les huiles végétales et extraits végétaux, en particulier les produits connus pour leur intérêt cosmétique, les colorants réactifs ou non ainsi que les dispersions de pigments, les filtres UV, les vitamines et molécules médicalement actives, les parfums huiles essentielles et arômes, les insecticides et répulsifs, les catalyseurs, les matériaux à changement de phase, les composés phénoliques, les « colorformers ».

La suspension ou dispersion aqueuse finale de microcapsules contient en général de 30 à 40% en poids d'actif ; elle peut être diluée, concentrée par les moyens habituels, voire séchée en une poudre pulvérulente.

La présente invention concerne également les microcapsules fabriquées par le procédé décrit ci-dessus.

Ces microcapsules à réservoir renfermant un principe actif lipophile, préparées au moyen du procédé ci-dessus, comprennent une enveloppe formée d'au moins deux polymères liés entre eux par des liaisons polaires, hydrogène ou covalentes formant la paroi desdites microcapsules le premier polymère, dénommé polymère interne, étant un copolymère silicone/mélamine/uréthane et le second polymère, dénommé polymère externe, étant un coacervat réticulé à base de polymère de gélatine et/ou de protéine végétale, et d'un polyacide.

De manière avantageuse ledit polymère externe représente entre 15 et 65 % en poids, de préférence entre 30 et 60% en poids, de la paroi desdites microcapsules. La fraction de matière d'origine naturelle de ces microcapsules permet de leur conférer une meilleure biodégradabilité que les microcapsules de l'art antérieur constituées exclusivement de molécules de synthèse.

La perméabilité des microcapsules selon l'invention peut être modulée en jouant sur les conditions de polymérisation de la paroi, ainsi que sur les caractéristiques dimensionnelles des microcapsules dont le diamètre peut varier entre 2 et 50µm, préférentiellement entre 5 et 20 micromètres.

Enfin il est avantageux de pouvoir faire varier les proportions du polymère biodégradable utilisé pour la coacervation complexe (polymère externe) ainsi que celle du polymère synthétique interne, car les deux phases sont préparées séparément au départ.

Ainsi plus le rapport polymère biodégradable/polymère synthétique sera grand et plus les microcapsules obtenues seront biodégradables et utilisables par exemple dans l'industrie cosmétique, la gélatine d'origine animale étant alors remplacée par une protéine d'origine végétale.

A l'inverse, un rapport polymère biodégradable/polymère synthétique diminué entrainera une plus grande résistance des microcapsules dans les milieux ou elles sont destinées à être utilisées.

De plus la proportion pondérale paroi/principe actif des microcapsules peut varier dans de grandes proportions, par exemple entre 5 et 40%, préférentiellement entre 7 et 25%.

Les microcapsules selon l'invention renferment avantageusement en tant que principe actif une molécule odorante, tel qu'un parfum.

La présente invention concerne également l'utilisation de ces microcapsules, notamment dans des formulations contenant des tensio-actifs.

Plus particulièrement ces microcapsules peuvent être utilisées dans des lessives liquides, des lessives en poudre, des détergents ménagers et industriels ou des adoucissants textiles.

Dans le domaine cosmétique, ces microcapsules peuvent être utilisées dans des shampoings, des produits de conditionnement pour cheveux, des dentifrices, des savons liquides, des nettoyants corporels ou des lotions. Les principes actifs peuvent alors être par exemple des filtres UV, des vitamines, des huiles insaturées, des actifs lipophiles pouvant renfermer des colorants ou des peptides.

Les microcapsules préparées selon le procédé de la présente invention peuvent aussi être utilisées dans beaucoup d'autres domaines, tels que l'industrie du papier (papier autocopiant de type NCR, papiers de sécurité), dans l'industrie textile (cosméto-textile, parfums, matériaux à changement de phase, mouchoirs, lingettes) de la publicité (publicités parfumées par exemple), l'industrie du cuir, la pharmacie, la médecine, l'industrie vétérinaire, les adhésifs, peintures et enduits, le bâtiment, sans que cette liste soit limitative.

### EXEMPLES

### Exemple 1 : Microcapsules renfermant un parfum préparées par le procédé selon la présente invention

a) Dans un bécher de 250 ml agité magnétiquement par un barreau de 45mm sont introduits successivement à température ambiante (25 °C) :
   - 140 g de parfum floral mX d'Iberchem
   - 12,2 g de polysilicate d'éthyle (TES 40 Wacker)
   - 6,7 g de tris-[3-(triméthoxysilyl)propyl]-isocyanurate (Geniosil GF 69 de wacker)
   - 2,92 g d'isocyanurate d'hexaméthylene diisocyanate (tolonate HDT-LV2 de Perstorp)
   - 5,2 g de résine mélamine carbamate (Cymel 2000A de Allnex)
   - 2,9 g de 3-mercaptopropyltriméthoxysilane (JH-S189 de JHSi).
b) Par ailleurs est préparée une solution dans un réacteur à double paroi de 1L agité par une hélice 4 pales de diamètre 7cm et chauffé à 50°C comme suit :
   - 80 g d'eau du robinet à 50°C
   - 2,25 g de carboxyméthylcellulose (Wallocel CT 35GA de Dow).
c) Dans un bain marie régulé à 43°C est placé un bécher de 600 ml, agité par une turbine de diamètre 6,5 cm. On introduit pour dissolution successivement :
   - 141 g d'eau du robinet
   - 0,33 g d'hydroxyéthylcellulose (250m d'Aqualon)
   - 7,0 g de gélatine 140 bloom (PBG03 de Tessenderlo)
   - 1,67 g d'acide nitrique à 20%
   - 2,8 g de sel de sodium d'un copolymère d'acide acrylique et méthacrylique (Synthran 8521 d'Interpolymer) mis préalablement à pH 4,5 avec de la soude à 50%.
d) La solution dans le parfum préparée au début est alors versée dans ce bécher et émulsionnée à 42°C pendant 30 mn, la vitesse de l'agitateur étant réglée pour obtenir un diamètre moyen de 10 à 12µm (vitesse entre 1000 et 1400 tr/min).
e) Le contenu du bécher est alors versé dans le réacteur contenant la solution de carboxyméthylcellulose puis le tout est refroidi à 8°C en 2h30, la vitesse de celui-ci étant augmentée ensuite pour éviter les gélifications qui peuvent éventuellement se former sur les bords du réacteur. 4,0 g de glutaraldéhyde à 50% dans l'eau sont alors ajoutés. La température est maintenue encore à 8°C pendant 7h.
f) Puis on chauffe à 50°C pendant 3h.
g) Enfin on revient à température ambiante et on ajuste le pH à 5,5 par de la soude. On ajoute ensuite épaississants, conservateurs, agents de déposition etc...

### Exemple 2 (comparatif) : Microcapsules obtenues par coacervation complexe standard

Les opérations précédentes sont répétées à l'identique, mais le parfum de la solution a) de l'exemple 1 précédent est utilisé seul, sans autre produit dissous au préalable.

### Comparaison des résultats :

Les microcapsules des exemples 1 et 2 sont comparées dans un adoucissant textile.

Les caractéristiques des microcapsules préparées sont regroupées dans le tableau 1 ci-après :

**[Tableau 1]**

| Type | Référence interne | Diamètre moyen | Formaldéhyde ppm |
|---|---|---|---|
| **Exemple 2** | 9027 | 13,5 µm | 0 |
| **Exemple 1** | 9087 | 11,3 µm | 0 |

### Mode opératoire :

Les microcapsules à 35% en poids de parfum sont incorporées à l'adoucissant textile standard du commerce non parfumé dans un rapport pondéral de 2%, mélangées à l'aide d'un agitateur sous forte agitation pendant 15 mn.

Chacun des mélanges est observé à l'œil nu, puis au microscope et sa stabilité est suivie dans le temps.

### Résultats :

Après observation au microscope et prise de photographie, les observations relatives aux différents mélanges, aussitôt après l'incorporation à la lessive, sont regroupées dans le tableau 2 ci-après :

**[Tableau 2]**

| **Microcapsules** | **Dans l'adoucissant textile** |
|---|---|
| Exemple 1 | Assez bien dispersées |
| Exemple 2 (comparatif) | Assez bien dispersées mais déformées |

10 Jours après un vieillissement accéléré à 50°C, on observe à nouveau les mélanges et on évalue l'intensité olfactive : plus celle-ci est importante, plus les microcapsules ont souffert (voir tableau 3 ci-dessous) :

**[Tableau 3]**

| **Micro capsules** | **Aspect de l'adoucissant textile** | **Observation au microscope** | **Intensité du parfum dans l'adoucissant** | **Intensité du parfum sur le tissu frotté** |
|---|---|---|---|---|
| Exemple 1 | Couleur blanc beige bien homogène | Dispersion correcte | Odeur très faible | Bonne odeur assez forte |
| Exemple 2 (comparatif) | épais | Assez bien dispersées | Odeur très forte | Odeur très faible |
| | Couleur beige | Mais très déformées | | |

### Conclusion

Les microcapsules à double paroi sont les plus étanches, elles relarguent moins de parfum que les autres microcapsules car elles ont été moins attaquées par les tensio-actifs présents dans l'adoucissant textile.

Les microcapsules obtenues par uniquement la coacervation complexe sont celles qui dégagent le plus d'odeur dans l'adoucissant textile et qui sont donc devenues les plus poreuses, ce qui est confirmé par l'odeur très faible des tissus frottés après 10 jours.

## Revendications

1. Procédé de fabrication de microcapsules à réservoir, renfermant un principe actif lipophile dans une enveloppe à base d'au moins deux polymères liés de manière covalente formant la paroi desdites microcapsules, le procédé comprenant les étapes suivantes :
(i) ajout à une phase lipophile, renfermant au moins un principe actif, d'au moins un monomère ou oligomère silane et/ou de silicates, d'au moins une résine mélamine et d'au moins un isocyanate, pour former un mélange A,
(ii) dispersion sous agitation du mélange A obtenu à l'étape (i) dans une phase continue aqueuse B à pH acide renfermant au moins une gélatine et/ou au moins une protéine végétale hydrosoluble, et au moins un polyacide, de manière à former par coacervation complexe une émulsion ou une dispersion de gouttelettes de type huile dans l'eau, enveloppées d'un coacervat, et à initier la formation d'un premier copolymère silicone/ mélamine/ polyuréthane enfermant ledit principe actif,
(iii) introduction éventuelle d'un colloïde protecteur de type dérivé de cellulose dans la phase continue aqueuse contenant la dispersion de gouttelettes,
(iv) ajout, à la dispersion de gouttelettes, à une température inférieure ou égale à 10°C, d'au moins un agent de réticulation du coacervat, permettant la co-réticulation, à l'interface des dites gouttelettes, du coacervat à base de gélatine et/ou de protéine végétale avec le premier copolymère silicone/mélamine/polyuréthane en formation, conduisant à des polymères co-réticulés, constituant la paroi desdites microcapsules en enfermant ledit principe actif,
(v) obtention d'une suspension aqueuse de microcapsules enfermant le principe actif lipophile.

2. Procédé selon la revendication 1, **caractérisé en ce que** le polyacide
de la phase aqueuse B comprend un polyacide de type poly(méth)acrylique ou polyaspartique et une carboxyalkylcellulose, de préférence la
carboxyméthylcellulose.

3. Procédé selon l'une des revendications 1 ou 2, **caractérisé en ce que** les proportions pondérales des constituants destinés à former le copolymère silicone/mélamine/polyuréthane, introduits dans le mélange A sont respectivement de 50 à 80% du monomère ou oligomère silane et/ou silicate, de 25 à 10% de la résine mélamine et de 25 à 10% de l'isocyanate, exprimées en poids sec du poids total desdits constituants.

4. Procédé selon la revendication 2, **caractérisé en ce que** la phase aqueuse B renferme les proportions pondérales suivantes : 50 à 80 % de gélatine et/ou de protéine végétale, 10 à 30 % de carboxyalkylcellulose, et 5 à 20 % de polyacide de type poly(méth)acrylique ou polyaspartique, exprimées en % du poids sec de coacervat.

5. Procédé selon l'une quelconque des revendications précédentes, **caractérisé en ce que** le ou les composés silanes et/ou silicates est/sont choisi(s) parmi les composés de formule (I) ou (II) ci-après : 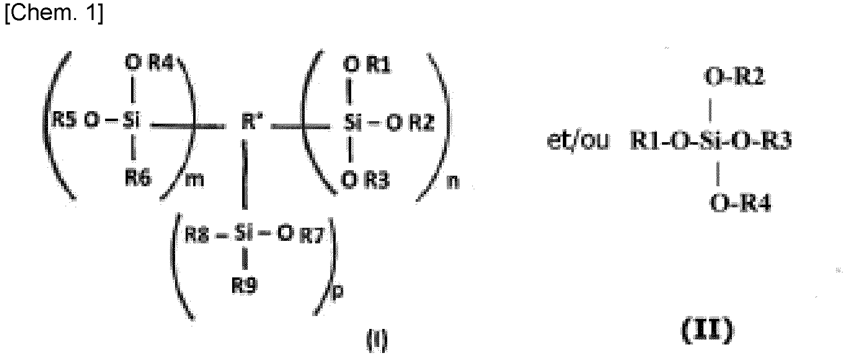
dans lesquels R1, R2, R3, R4, R5, R6, R7, R8, R9 sont des radicaux alkyle, linéaires ou cycliques, substitués ou non,
R° est une molécule organique et/ou silicone,
les groupes entre ( ) étant reliés à R° par un atome de silicium et sont présents m, n, ou p fois, et m, n, p pouvant être nuis individuellement, mais la somme m+n+p étant au moins égale à 1.

6. Procédé selon la revendication 5, **caractérisé en ce que** le composé de formule (II) est choisi parmi le polysilicate de méthyle, le polysilicate d'éthyle ou un mélange de ceux-ci.

7. Procédé selon l'une des revendications précédentes, **caractérisé en ce que** la mélamine est choisie parmi une mélamine-aldéhyde ou une mélamine-carbamate liposoluble, et est de préférence une mélamine-carbamate.

8. Procédé selon l'une des revendications précédentes, **caractérisé en ce que** l'isocyanate est choisi parmi : les diisocyanates de toluène TDI, d'hexaméthylène HDI, de diphénylméthylène MDI, ou d'isophorone IPDI, les dimères ou trimères du diisocyanate d'hexaméthylène tel que l'isocyanurate d'hexaméthylène, les uretdione, ou uretonimine, ou plusieurs de ceux-ci.

9. Procédé selon l'une des revendications précédentes, **caractérisé en ce que** l'étape (ii) s'effectue à un pH compris entre 3,0 et 5,5, de préférence compris entre 3,5 et 4,5, par ajout dans la phase aqueuse d'au moins un acide comprenant l'acide nitrique.

10. Procédé selon l'une des revendications 2 à 9, **caractérisé en ce que** l'étape (ii) est réalisée en deux temps :
- dans un premier temps dispersion du mélange A obtenu à l'étape (i) dans une phase continue aqueuse B à pH acide renfermant au moins une gélatine et/ou au moins une protéine végétale hydrosoluble, au moins un polyacide de type poly(méth)acrylique,
- puis dans un second temps ajout d'une solution aqueuse de carboxyalkylcellulose à
la dispersion obtenue dans le premier temps.

11. Procédé selon l'une des revendications précédentes, **caractérisé en ce que** l'agent de réticulation introduit à l'étape (iv) comprend le glutaraldéhyde.

12. Procédé selon la revendication 11, **caractérisé en ce que** l'étape (i) est réalisée à température ambiante entre 15-25 °C, l'étape (ii) à une température comprise entre 40° et 50 °C, l'émulsion formée étant ensuite refroidie à une température comprise entre 7°C et 10 °C, puis est ajouté le glutaraldéhyde et cette température est maintenue pendant au moins 4 heures, avant que ne soit terminée la polymérisation à chaud entre 40°C et 80°C pendant 1 à 6h.

13. Microcapsules à réservoir renfermant un principe actif lipophile, préparées au moyen du procédé selon l'une quelconque des revendications précédentes, comprenant une enveloppe formée d'au moins deux polymères liés entre eux par des liaisons covalentes formant la paroi desdites microcapsules le premier polymère, dénommé polymère interne, étant un copolymère silicone/mélamine/uréthane et le second polymère, dénommé polymère externe, étant un coacervat réticulé à base de polymère de gélatine et/ou de protéine végétale, et d'un polyacide.

14. Microcapsules selon la revendication 13, **caractérisées en ce que** ledit polymère externe, représente entre 15 et 65 % en poids, de préférence entre 30 et 60 % en poids, de la paroi desdites microcapsules.

15. Microcapsules selon la revendication 13, renfermant en tant que principe actif une molécule odorante, tel qu'un parfum.

16. Utilisation des microcapsules préparées au moyen du procédé selon l'une quelconque des revendications 1 à 12 dans des formulations contenant des tensio-actifs.

17. Utilisation des microcapsules préparées au moyen du procédé selon l'une quelconque des revendications 1 à 12 dans des lessives liquides, des lessives en poudre, des détergents ménagers et industriels ou des adoucissants textiles.

18. Utilisation des microcapsules préparées au moyen du procédé selon l'une quelconque des revendications 1 à 12 dans des shampoings, des produits de conditionnement pour cheveux, des dentifrices, des savons liquides, des nettoyants corporels ou des lotions.

## Patentansprüche

1. Verfahren zur Herstellung von Mikrokapseln mit Behälter, die einen lipophilen Wirkstoff enthalten, in einer Hülle auf der Grundlage von mindestens zwei kovalent gebundenen Polymeren, die die Wand der Mikrokapseln bilden, wobei das Verfahren die folgenden Schritte umfasst:
(i) Hinzufügen zu einer lipophilen Phase, die mindestens einen Wirkstoff enthält, von mindestens einem Silanmonomer oder -oligomer und/oder von Silikaten, von mindestens einem Melaminharz und von mindestens einem Isocyanat, um ein Gemisch A zu bilden,
(ii) Dispergieren, unter Rühren, des Gemischs A, erhalten in Schritt (i), in einer kontinuierlichen wässrigen Phase B mit einem sauren pH, der mindestens eine Gelatine und/oder mindestens ein wasserlösliches pflanzlichen Protein und mindestens eine Polysäure enthält, um durch eine komplexe Koazervation eine Emulsion oder eine Dispersion von Tröpfchen vom Typ Öl-in-Wasser, umhüllt von einem Koazervat, zu bilden, und um die Bildung eines ersten Silikon-/Melamin-/Polyurethancopolymers zu initiieren, das den Wirkstoff einschließt,
(iii) eventuelles Einführen eines Schutzkolloids vom Typ Cellulosederivat in die kontinuierliche wässrige Phase, die die Dispersion von Tröpfchen enthält,
(iv) Hinzufügen, zur Dispersion von Tröpfchen, bei einer Temperatur von weniger als oder gleich wie 10 °C, von mindestens einem Vernetzungsmittel des Koazervats, das die Co-Vernetzung an der Schnittstelle der Tröpfchen des Koazervats auf der Grundlage von Gelatine und/oder von pflanzlichem Protein mit dem ersten Silikon-/Melamin-/Polyurethancopolymer in Bildung ermöglicht, was zu co-vernetzten Polymeren führt, die die Wand der Mikrokapseln darstellen, indem sie den Wirkstoff einschließen.
(v) Erhalten einer wässrigen Suspension von Mikrokapseln, die den Wirkstoff einschließen.

2. Verfahren nach Anspruch 1, **dadurch gekennzeichnet, dass** die Polysäure der wässrigen Phase B eine Polysäure vom Typ Poly(meth)acryl oder Polyasparagin und eine Carboxyalkylcellulose, vorzugsweise Carboxymethylcellulose, umfasst.

3. Verfahren nach einem der Ansprüche 1 oder 2, **dadurch gekennzeichnet, dass** die Gewichtsanteile der Bestandteile, die dazu ausgelegt sind, das Silikon-/Melamin-/Polyurethancopolymer zu bilden, die in das Gemisch A eingeführt wurden, jeweils 50 bis 80 % des Silanmonomers oder -oligomers und/oder Silikats, von 25 bis 10 % des Melaminharzes und von 25 bis 10 % des Isocyanats sind, ausgedrückt in Trockengewicht des Gesamtgewichts der Bestandteile.

4. Verfahren nach Anspruch 2, **dadurch gekennzeichnet, dass** die wässrige Phase B die folgenden Gewichtsproportionen enthält: 50 bis 80 % Gelatine und/oder pflanzliches Protein, 10 bis 30 % Carboxyalkylcellulose und 5 bis 20 % Polysäure vom Typ Poly(meth)acryl oder Polyasparagin, ausgedrückt in Prozent des Trockengewichts von Koazervat.

5. Verfahren nach einem der vorhergehenden Ansprüche, **dadurch gekennzeichnet, dass** die Silan- und/oder Silikatzusammensetzung(en) ausgewählt ist/sind aus den Verbindungen mit der folgenden Formel (I) oder (II): 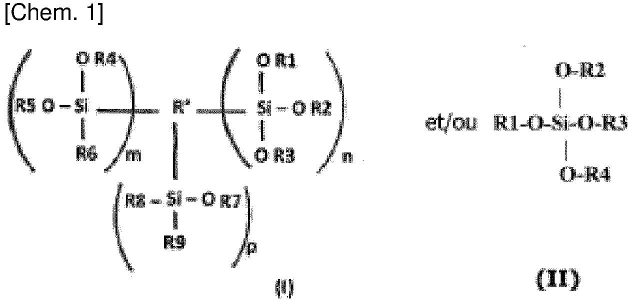
wobei R1, R2, R3, R4, R5, R6, R7, R8, R9 lineare oder zyklische, subsituierte oder nicht substituierte Alkylradikale sind,
R° ein organisches Molekül und/oder Silikon ist,
die Gruppen zwischen ( ) mit R° durch ein Siliziumatom verbunden sind und m-, n-, oder p-mal vorhanden sind, und wobei m, n, p individuell null sein können, wobei jedoch die Summe m+n+p mindestens gleich 1 ist.

6. Verfahren nach Anspruch 5, **dadurch gekennzeichnet, dass** die Verbindung mit der Formel (II) ausgewählt ist aus Methylpolysilicat, Ethylpolysilikat und einem Gemisch derselben.

7. Verfahren nach einem der vorhergehenden Ansprüche, **dadurch gekennzeichnet, dass** das Melamin ausgewählt ist aus einem Melaminaldehyd oder einem fettlöslichen Melamincarbamat und vorzugsweise ein Melamincarbamat ist.

8. Verfahren nach einem der vorhergehenden Ansprüche, **dadurch gekennzeichnet, dass** das Isocyanat ausgewählt ist aus: Toluendiisocyanaten TDI, Hexamethylen HDI, Diphenylmethylen MDI oder Isophoron IPDI, den Dimeren oder Trimeren des Hexamethylendiisocyanats wie Hexamethylenisocyanurat, Uretdion oder Uretonimin oder mehreren derselben.

9. Verfahren nach einem der vorhergehenden Ansprüche, **dadurch gekennzeichnet, dass** Schritt (ii) bei einem pH durchgeführt wird, der im Bereich zwischen 3,0 und 5,5 liegt, vorzugsweise im Bereich zwischen 3,5 und 4,5 mm, durch Hinzufügen in die wässrige Phase von mindestens einer Säure, umfassend Salpetersäure.

10. Verfahren nach einem der Ansprüche 2 bis 9, **dadurch gekennzeichnet, dass** Schritt (ii) in zwei Durchgängen durchgeführt wird:
- in einem ersten Durchgang Dispergieren des Gemischs A, erhalten in Schritt (i), in einer kontinuierlichen wässrigen Phase B mit einem sauren pH, die mindestens eine Gelatine und/oder mindestens ein wasserlösliches pflanzlichen Protein enthält, mindestens einer Polysäure vom Typ Poly(meth)acryl,
- dann, in einem zweiten Durchgang, Hinzufügen einer wässrigen Lösung von Carboxyalkylcellulose zur Dispersion, erhalten im ersten Durchgang.

11. Verfahren nach einem der vorhergehenden Ansprüche, **dadurch gekennzeichnet, dass** das Vernetzungsmittel, eingeführt in Schritt (iv), Glutaraldehyd umfasst.

12. Verfahren nach Anspruch 11, **dadurch gekennzeichnet, dass** Schritt (i) bei einer Umgebungstemperatur zwischen 15 und 25 °C durchgeführt wird, Schritt (ii) bei einer Temperatur, die im Bereich zwischen 40° und 50 °C liegt, wobei die gebildete Emulsion anschließend auf eine Temperatur abgekühlt wird, die im Bereich zwischen 7 °C und 10 °C liegt, dann das Glutaraldehyd hinzugefügt wird und diese Temperatur während mindestens 4 Stunden beibehalten wird, bevor die Heißpolymerisierung zwischen 40 °C und 80 °C während 1 bis 6h beendet ist.

13. Mikrokapseln mit Behälter, die einen lipophilen Wirkstoff enthalten, der mit Hilfe des Verfahrens nach einem der vorhergehenden Ansprüche zubereitet wurde, umfassend eine Hülle, die aus mindestens zwei Polymeren gebildet ist, die untereinander durch kovalente Bindungen gebunden sind, die die Wand der Mikrokapseln bilden, wobei das erste Polymer, bezeichnet als internes Polymer, ein Silikon-/Melamin-/Urethancopolymer ist und das zweite Polymer, bezeichnet als externes Polymer, ein vernetztes Koazervat auf der Grundlage von Gelatine und/oder pflanzlichem Protein und einer Polysäure ist.

14. Mikrokapseln nach Anspruch 13, **dadurch gekennzeichnet, dass** das externe Polymer zwischen 15 und 65 Gewichtsprozent, vorzugsweise zwischen 30 und 60 Gewichtsprozent der Wand der Mikrokapseln darstellt.

15. Mikrokapseln nach Anspruch 13, die als Wirkstoff ein Duftmolekül wie ein Parfüm enthalten.

16. Verwendung der Mikrokapseln, zubereitet mit Hilfe des Verfahrens nach einem der Ansprüche 1 bis 12, in Formulierungen, die Tenside enthalten.

17. Verwendung der Mikrokapseln, zubereitet mit Hilfe des Verfahrens nach einem der Ansprüche 1 bis 12, in flüssigen Waschmitteln, pulverförmigen Waschmitteln, Haushalts- und industriellen Reinigungsmitteln oder Textilweichmachern.

18. Verwendung der Mikrokapseln, zubereitet mit Hilfe des Verfahrens nach einem der Ansprüche 1 bis 12, in Shampoos, Haarkonditionierungsprodukten, Zahnpasten, Flüssigseifen, Körperreinigern oder Lotionen.

## Claims

1. A process for manufacturing reservoir-type microcapsules, containing a lipophilic active principle in a shell based on at least two covalently bonded polymers forming the wall of said microcapsules, the process comprising the following steps:
(i) addition to a lipophilic phase, containing at least one active principle, of at least one silane and/or silicate monomer or oligomer, at least one melamine resin and at least one isocyanate, to form a mixture A,
(ii) dispersion with stirring of the mixture A obtained in step (i) in an aqueous continuous phase B at acidic pH containing at least one gelatin and/or at least one water-soluble plant protein, and at least one polyacid, so as to form, by complex coacervation, an emulsion or a dispersion of droplets of oil-in-water type, enveloped by a coacervate, and to initiate the formation of a first silicone/melamine/polyurethane copolymer enclosing said active principle,
(iii) optional introduction of a protective colloid such as a cellulose derivative into the aqueous continuous phase containing the dispersion of droplets,
(iv) addition, to the dispersion of droplets, at a temperature of less than or equal to 10°C, of at least one coacervate-crosslinking agent, enabling the co-crosslinking, at the interface of said droplets, of the gelatin-based and/or plant protein-based coacervate with the first silicone/melamine/polyurethane copolymer undergoing formation, leading to co-crosslinked polymers constituting the wall of said microcapsules and enclosing said active principle,
(v) production of an aqueous suspension of microcapsules enclosing the lipophilic active principle.

2. The process as claimed in claim 1, **characterized in that** the polyacid of the aqueous phase B comprises a polyacid of poly(meth)acrylic or polyaspartic type and a carboxyalkylcellulose, preferably carboxymethylcellulose.

3. The process as claimed in either of claims 1 and 2, **characterized in that** the weight proportions of the constituents intended to form the silicone/melamine/polyurethane copolymer, introduced into the mixture A, are, respectively, from 50% to 80% of the silane and/or silicate monomer or oligomer, from 25% to 10% of the melamine resin and from 25% to 10% of the isocyanate, expressed as dry weight relative to the total weight of said constituents.

4. The process as claimed in claim 2, **characterized in that** the aqueous phase B contains the following weight proportions: 50% to 80% of gelatin and/or plant protein, 10% to 30% of carboxyalkylcellulose, and 5% to 20% of polyacid of poly(meth)acrylic or polyaspartic type, expressed as a percentage of the dry weight of coacervate.

5. The process as claimed in any one of the preceding claims, **characterized in that** the silane compound(s) are chosen from the compounds of formula (I) or (II) below: 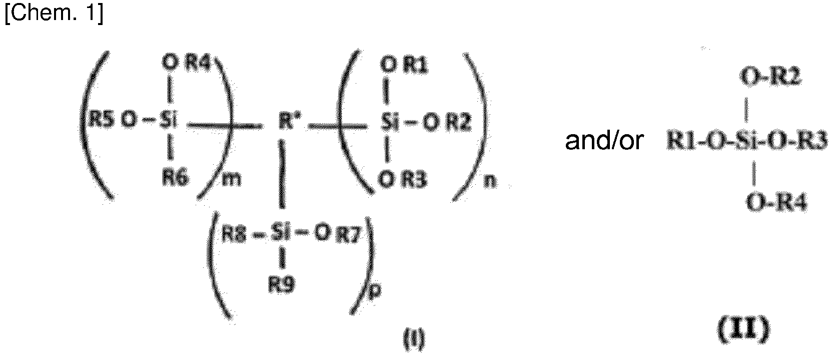
in which R1, R2, R3, R4, R5, R6, R7, R8 and R9 are substituted or unsubstituted, linear or cyclic alkyl radicals,
R° is an organic and/or silicone molecule,
the groups between ( ) being linked to R° via a silicon atom and are present m, n or p times, and m, n, p possibly being individually zero, but the sum m+n+p being at least equal to 1.

6. The process as claimed in claim 5, **characterized in that** the compound of formula (II) is chosen from methyl polysilicate, ethyl polysilicate or a mixture thereof.

7. The process as claimed in one of the preceding claims, **characterized in that** the melamine is chosen from a liposoluble melamine-aldehyde or melamine-carbamate, and is preferably a melamine-carbamate.

8. The process as claimed in one of the preceding claims, **characterized in that** the isocyanate is chosen from: toluene diisocyanate TDI, hexamethylene diisocyanate HDI, diphenylmethylene diisocyanate MDI, or isophorone diisocyanate IPDI, hexamethylene diisocyanate dimers or trimers, such as hexamethylene isocyanurate, uretdione, or uretonimine, or several thereof.

9. The process as claimed in one of the preceding claims, **characterized in that** step (ii) is performed at a pH of between 3.0 and 5.5, preferably between 3.5 and 4.5, by adding to the aqueous phase at least one acid comprising nitric acid.

10. The process as claimed in one of claims 2 to 9, **characterized in that** step (ii) is performed in two stages:
- in a first stage, dispersion of the mixture A obtained in step (i) in an aqueous continuous phase B at acidic pH containing at least one gelatin and/or at least one water-soluble plant protein, at least one polyacid of poly(meth)acrylic type,
- and then, in a second stage, addition of an aqueous solution of carboxyalkylcellulose to the dispersion obtained in the first stage.

11. The process as claimed in one of the preceding claims, **characterized in that** the crosslinking agent introduced into step (iv) comprises glutaraldehyde.

12. The process as claimed in claim 11, **characterized in that** step (i) is performed at room temperature (15-25 °C), step (ii) at a temperature of between 40°C and 50°C, the emulsion formed being subsequently cooled to a temperature of between 7°C and 10°C, the glutaraldehyde is then added and this temperature is maintained for at least 4 hours, before completing the hot polymerization between 40°C and 80°C for 1 to 6 hours.

13. A reservoir-type microcapsule containing a lipophilic active principle, prepared by means of the process as claimed in any one of the preceding claims, comprising a shell formed from at least two polymers bonded together by covalent bonds forming the wall of said microcapsules, the first polymer, referred to as the internal polymer, being a silicone/melamine/urethane copolymer and the second polymer, referred to as the external polymer, being a crosslinked coacervate based on a gelatin polymer and/or plant protein, and a polyacid.

14. The microcapsule as claimed in claim 13, **characterized in that** said outer polymer represents between 15% and 65% by weight and preferably between 30% and 60% by weight of the wall of said microcapsules.

15. The microcapsule as claimed in claim 13, containing, as active principle, an odorous molecule, such as a fragrance.

16. The use of the microcapsule prepared by means of the process as claimed in any one of claims 1 to 12 in formulations containing surfactants.

17. The use of the microcapsule prepared by means of the process as claimed in any one of claims 1 to 12, in liquid washing products, washing powders, household and industrial detergents or fabric softeners.

18. The use of the microcapsule prepared by means of the process as claimed in any one of claims 1 to 12, in shampoos, hair-conditioning products, toothpastes, liquid soaps, body cleansers or lotions.
